# EUROPEAN PATENT SPECIFICATION

(11) **EP 3 134 532 B1**
(45) Date of publication and mention of the grant of the patent: **17.04.2019**
(21) Application number: 15718853.3
(22) Date of filing: 22.04.2015
(51) Int. Cl.: C12N 15/873, C12N 15/877, A01K 67/027

(54) **IMPROVED METHOD FOR RECONSTRUCTING A NON-HUMAN ANIMAL EMBRYO**
VERFAHREN ZUR REKONSTRUKTION EINES NICHT-MENSCHLICHEN TIERISCHEN EMBRYOS
PROCÉDÉ AMÉLIORÉ DE RECONSTRUCTION D'UN EMBRYON ANIMAL NON HUMAIN

(30) Priority: 22.04.2014 US 201461982357 P
(43) Date of publication of application: 01.03.2017
(73) Proprietor: Universita' Degli Studi di Teramo, 64100 Teramo (TE) (IT)
(72) Inventor: LOI, Pasqualino, 64020 Nepezzano (TE) (IT); IUSO, Domenico, 71122 Foggia (FG) (IT); CZERNIK, Marta, 64011 Alba Adriatica (TE) (IT); ZACCHINI, Federica, 64100 Teramo (TE) (IT); PTAK, Grazyna, 64020 Nepezzano (TE) (IT); KHOCHBIN, Saadi, 38706 La Tronche, Grenoble (FR); FIDANZA, Antonella, 64100 Teramo (TE) (IT)
(74) Representative: Capasso, Olga
(86) International application number: PCT/EP2015/058701
(87) International publication number: WO 2015/162170

(56) References cited:
- WO-A1-00/74477
- P Loi: "Improving the efficiency of somatic cell nuclear transfer (SCNT).", , 5 December 2011 (2011-12-05), pages 1-2, XP055197483, Retrieved from the Internet: URL:http://www.researchvalue.net/repositor y/result.html?id=749 [retrieved on 2015-06-22]
- ROH S: "Porcine nuclear transfer using somatic donor cells altered to expressmale germ cell function", REPRODUCTION, FERTILITY, AND DEVELOPMENT AUSTRALIA, vol. 21, 1 January 2009 (2009-01-01), pages 882-891, XP009184978,
- MARTA TEPEREK ET AL: "Nuclear reprogramming of sperm and somatic nuclei in eggs and oocytes", REPRODUCTIVE MEDICINE AND BIOLOGY, vol. 12, no. 4, 4 June 2013 (2013-06-04), pages 133-149, XP055197268, ISSN: 1445-5781, DOI: 10.1007/s12522-013-0155-z
- LOI P ET AL: "Genetic rescue of an endangered mammal by cross-species nuclear transfer using post-mortem somatic cells", NATURE BIOTECHNOLOGY, NATURE PUBLISHING GROUP, US, vol. 19, 1 October 2001 (2001-10-01), pages 962-964, XP002966779, ISSN: 1087-0156, DOI: 10.1038/NBT1001-962
- IUSO A. ET AL.: "Conversion of the chromatin of somatic cells into spermatid-like structures", REPRODUCTION, FERTILITY AND DEVELOPMENT, vol. 27, no. 1, 42, 4 December 2014 (2014-12-04), page 114, XP009185023,
- A. Yabuuchi ET AL: "Nuclear transfer of mouse follicular epithelial cells pretreated with spermine, protamine, or putrescine", Journal of Experimental Zoology, vol. 289, no. 3, 1 January 2001 (2001-01-01), pages 208-212, XP055197527, ISSN: 0022-104X, DOI: 10.1002/1097-010X(20010215)289:3<208::AID- JEZ8>3.0.CO;2-R

### SEQUENCE LISTING

<110> Loi, Pasqualino Iuso, Domenico Czernik, Marta Zacchini, Federica Fidanza, Antonella Khochbin, Saadi Ptak, Grazyna
<120> IMPROVED METHOD FOR RECONSTRUCTING A NON-HUMAN ANIMAL EMBRYO
<130> PCT 126536
<150> US61/982,357
   <151> 2014-04-22
<160> 10
<170> PatentIn version 3.5
<210> 1
   <211> 426
   <212> DNA
   <213> Homo sapiens
<400> 1
   gactcacagc ccacagagtt ccacctgctc acaggttggc tggctcagcc aaggtggtgc      60
   cctgctctga gcattcaggc caagcccatc ctgcaccatg gccaggtaca gatgctgtcg     120
   cagccagagc cggagcagat attaccgcca gagacaaaga agtcgcagac gaaggaggcg     180
   gagctgccag acacggagga gagccatgag gtgctgccgc cccaggtaca gaccgcgatg     240
   tagaagacac taattgcaca aaatagcaca tccaccaaac tcctgcctga gaatgttacc     300
   agacttcaag atcctcttgc cacatcttga aaatgccacc atccaataaa aatcaggagc     360
   ctgctaagga acaatgccgc ctgtcaataa atgttgaaaa gtcatcccaa aaaaaaaaaa     420
   aaaaaa                                                                426
<210> 2
   <211> 51
   <212> PRT
   <213> Homo sapiens
<400> 2
   Met Ala Arg Tyr Arg Cys Cys Arg Ser Gln Ser Arg Ser Arg Tyr Tyr
   1               5                   10                  15
   Arg Gln Arg Gln Arg Ser Arg Arg Arg Arg Arg Arg Ser Cys Gln Thr
               20                  25                  30
   Arg Arg Arg Ala Met Arg Cys Cys Arg Pro Arg Tyr Arg Pro Arg Cys
           35                  40                  45
   Arg Arg His
   50
<210> 3
   <211> 683
   <212> DNA
   <213> Homo sapiens
<400> 3
   agaccagacc aacagtaaca ccaagggcag gtgggcaggc ctccgccctc ctcccctact       60
   ccagggccca ctgcagcctc agcccaggag ccaccagatc tcccaacacc atggtccgat      120
   accgcgtgag gagcctgagc gaacgctcgc acgaggtgta caggcagcag ttgcatgggc      180
   aagagcaagg acaccacggc caagaggagc aagggctgag cccggagcac gtcgaggtct      240
   acgagaggac ccatggccag tctcactata ggcgcagaca ctgctctcga aggaggctgc      300
   accggatcca caggcggcag catcgctcct gcagaaggcg caaaagacgc tcctgcaggc      360
   accggaggag gcatcgcaga ggctgcagaa ccaggaagag aacatgcaga aggcactaag      420
   cttcctgggc ccctcacccc cagctggaaa ttaagaaaaa gtcgcccgaa acaccaagtg      480
   aggccatagc aattccccta catcaaatgc tcaagccccc agctggaagt taagagaaag      540
   tcacctgccc aagaaacacc gagtgaggcc atagcaactc ccctacatca aatgctcaag      600
   ccctgagttg ccgccgagaa gcccacaaga tctgagtgaa atgagcaaaa gtcacctgcc      660
   caataaagct tgacaagaca ctc                                              683
<210> 4
   <211> 102
   <212> PRT
   <213> Homo sapiens
<400> 4
   Met Val Arg Tyr Arg Val Arg Ser Leu Ser Glu Arg Ser His Glu Val
   1               5                   10                  15
   Tyr Arg Gln Gln Leu His Gly Gln Glu Gln Gly His His Gly Gln Glu
               20                  25                  30
   Glu Gln Gly Leu Ser Pro Glu His Val Glu Val Tyr Glu Arg Thr His
           35                  40                  45
   Gly Gln Ser His Tyr Arg Arg Arg His Cys Ser Arg Arg Arg Leu His
       50                  55                  60
   Arg Ile His Arg Arg Gln His Arg Ser Cys Arg Arg Arg Lys Arg Arg
   65                  70                  75                  80
   Ser Cys Arg His Arg Arg Arg His Arg Arg Gly Cys Arg Thr Arg Lys
                     85                  90                  95
   Arg Thr Cys Arg Arg His
               100
<210> 5
   <211> 415
   <212> DNA
   <213> Homo sapiens
<400> 5
   gcccctcatt ttggcagaac ttaccatgtc gaccagccgc aaattaaaga gtcatggcat       60
   gaggaggagc aagagccgat ctcctcacaa gggagtcaag agaggtggca gcaaaagaaa      120
   ataccgtaag ggcaacctga aaagtaggaa acggggcgat gacgccaatc gcaattaccg      180
   ctcccacttg tgagccccca gcgggctctg ccctggtgcg cttcacacag caccaagcag      240
   caacaagaac agcagaaggg gaactgccaa ggagacctga tgttagatca aagccagaga      300
   ggagcctatg gaatgtggat caaatgccag ttgtgacgaa atgaggaatg tatatgttgg      360
   ctgtttttcc ccaacatctc aataaaactt tgaaagcaga aaaaaaaaaa aaaaa           415
<210> 6
   <211> 55
   <212> PRT
   <213> Homo sapiens
<400> 6
   Met Ser Thr Ser Arg Lys Leu Lys Ser His Gly Met Arg Arg Ser Lys
   1               5                   10                  15
   Ser Arg Ser Pro His Lys Gly Val Lys Arg Gly Gly Ser Lys Arg Lys
               20                  25                  30
   Tyr Arg Lys Gly Asn Leu Lys Ser Arg Lys Arg Gly Asp Asp Ala Asn
           35                  40                  45
   Arg Asn Tyr Arg Ser His Leu
       50                  55
<210> 7
   <211> 599
   <212> DNA
   <213> Homo sapiens
<400> 7
   aggatgagga ggaggccctg cccctccaaa cgtggcctcc tatggacacc cagactcaca     60
   gccttcctat cacccacact cagctccata gcaactctca gccccaaagc cgcacctgca    120
   cccgccattg ccaaaccttc agccagagtt gcagacagag ccatcgtggc agccggagcc    180
   agagctccag ccagagcccg gccagccacc gcaacccaac tggagcccac agctcatccg    240
   gccaccagag ccagagtccc aacactagtc caccaccaaa gcgccacaaa aagactatga    300
   actcccacca ctctcccatg cggcccacca tcctgcactg ccgctgcccc aagaacagaa    360
   agaacttgga aggcaagctg aaaaagaaaa aaatggccaa gaggatccag caggtgtaca    420
   aaaccaagac gcggagctca ggatggaaat ccaactaatg agaccgcact ccttggcttg    480
   ttcctgcgtg tttcacccaa aggagaaaat gctaggatga agtcaatctt cttgcaggaa    540
   catgttacta tggtgatttc tacgcaacac taattaaagc ttgtacctgg aagactaaa     599
<210> 8
   <211> 138
   <212> PRT
   <213> Homo sapiens
<400> 8
   Met Asp Thr Gln Thr His Ser Leu Pro Ile Thr His Thr Gln Leu His
   1               5                   10                  15
   Ser Asn Ser Gln Pro Gln Ser Arg Thr Cys Thr Arg His Cys Gln Thr
               20                  25                  30
   Phe Ser Gln Ser Cys Arg Gln Ser His Arg Gly Ser Arg Ser Gln Ser
           35                  40                  45
   Ser Ser Gln Ser Pro Ala Ser His Arg Asn Pro Thr Gly Ala His Ser
       50                  55                  60
   Ser Ser Gly His Gln Ser Gln Ser Pro Asn Thr Ser Pro Pro Pro Lys
   65                  70                  75                  80
   Arg His Lys Lys Thr Met Asn Ser His His Ser Pro Met Arg Pro Thr
                   85                  90                  95
   Ile Leu His Cys Arg Cys Pro Lys Asn Arg Lys Asn Leu Glu Gly Lys
               100                 105                 110
   Leu Lys Lys Lys Lys Met Ala Lys Arg Ile Gln Gln Val Tyr Lys Thr
           115                 120                 125
   Lys Thr Arg Ser Ser Gly Trp Lys Ser Asn
       130                 135
<210> 9
   <211> 19
   <212> DNA
   <213> Artificial Sequence
<220>
   <223> Synthetic Primer
<400> 9
   atggccagat accgatgct 19
<210> 10
   <211> 18
   <212> DNA
   <213> Artificial Sequence
<220>
   <223> Synthetic Primer
<400> 10
   cagcatcttc gcctcctc 18

## Description

### FIELD OF THE INVENTION

The invention relates to cloning of non-human animal individuals by Somatic Cells Nuclear Transfer (SCNT). In particular the present invention refers to a method for reconstructing a non-human animal embryo and discloses a method for obtaining a non-human animal.

### BACKGROUND OF THE INVENTION

SCNT allows the asexual reproduction of individual by transplanting a somatic cell into an enucleated oocyte. SCNT has a tremendous potential as a reproductive technology for both domestic and wild animals. High productive or rare genotypes can be expanded through cloning (Wells D., 2003; Loi P., et al, 2001; Holt W., 2004); non-living animals can be "resuscitated" by nuclear transfer of non viable cells (Loi et al., 2001-2008; Wakayama et al., 2007), and finally, transgenic non-human animals could be produced at higher efficiency by nuclear transfer of genetically modified cells (Robl et al., 2007; Niemann and Kues 2007; Prather et al., 2003).

However, SCNT efficiency is currently very low, and even though there are differences between species, only 1-5% of offspring is usually obtained. The low efficiency of SCNT is a consequence of incomplete nuclear reprogramming of the somatic cell nucleus transplanted into the enucleated oocyte. Nuclear reprogramming refers to the elimination of chromatin modifications associated with cell differentiation by the enucleated oocyte.

Despite many years passed by since the production of the first clone (Wilmut et al., 1997), very little progress has been achieved in non-human animal cloning.

The reasoning behind SCNT failures is that whereas, oocyte reprogramming machinery perfectly fits with spermatozoa nuclei, they lack proper molecular tools to functionally interact with the complex chromatin organization of a somatic cell nucleus.

To improve SCNT, a radical modification of chromatin organization of a somatic cell before nuclear transfer should be induced, as suggested in prior publications (Loi & Cappai, 2000; Loi et al., 2002).

Loi P., "Improving the efficiency of somatic cell nuclear transfer (SCNT)", 2011 refers to the transfection of sheep fibroblasts with BRDT (testis specific bromodomain), resulting in chromatin condensation in 85% of the cells expressing BRDT.

Roh S. et al, Reproduction, Fertility and Development, 2009, 21, 882-891, refers to the permeabilization of a porcine fibroblast followed by incubation with testis extract (TE). Teperek M. et al., Reprod Med Biol (2013) 12: 133-149 reviews SCNT with emphasis on the reprogramming of somatic nuclei and consider the chromatin condensation as a complicated multi-step process.

Yabuuchi et al., Journal of experimental zoology, 289:208-212 (2001) refers to the pretreatment of a donor cell (mouse follicular epithelial cell) with protamine, spermine or putrescine in the cultivation medium. The pretreated donor cells are first fused with enucleated oocytes, and the nuclei from reconstituted eggs at the two-cell stage are fused with the enucleated fertilized two-cell embryos. The proportion of reconstituted embryos that developed into blastocysts are not significantly different among groups. After transfer to recipients, implantation rates are not different between groups and fetuses are obtained in protamine- and spermine- treated groups as well as in control groups. The International Application WO00/74477 refers to a process for reconstructing an animal embryo wherein the chromatin in the nucleus of a donor cell is subjected to denaturating conditions before transferring into the recipient cell. The strategies proposed in WO00/74477 for modifying the somatic chromatin is unspecific, and relies on the bulk effects of a thermal treatment to make it more easily reprogrammable by oocyte. Therefore, there is still the need to provide methods for specifically remodeling the chromatin organization of somatic cells, in order to improve SCNT.

### SUMMARY OF INVENTION

In the present invention, authors have found, for the very first time, that the transient expression of human protamine 1 (NCBI Reference Sequence: NM_002761.2 (SEQ ID NO:1) ; protein, GenBank: AAA63249.1 (SEQ ID NO:2), Rousseaux, et al., 2008; 2011) induces somatic cell nuclei to acquire a structural organization similar to that found in spermatozoa, the cell created for fertilization. The same effect might be elicited simply by inducing an uptake of short synthetic peptides corresponding to the functional part of protamine. Hence, by the process of the invention somatic cell nuclei acquire a spermatozoa like chromatin organization.

"Protaminized" nuclei are then injected into enucleated oocytes and physiologically remodeled by the oocyte's cytoplasm leading to a nuclear reprogramming to the blastocyst stage in high proportion.

Therefore the present invention induces an improvement in nuclear reprogramming in somatic cells nuclear transfer (SCNT), and consequently an improvement in its efficiency.

In the present invention, the modification of somatic chromatin is specific and targeted, for the induced protamine-dependent conformation, like spermatid / spermatozoa in nuclei of somatic cells (never achieved so far), mimics the physiological condition and matches perfectly the reprogramming machinery of the oocyte, resulting in an improved nuclear reprogramming.

Authors demonstrated that protaminized somatic cells (fibroblasts as exemplified in the present invention, but might be any kind of non-human animal cell) display in a repeatable manner (34 replicates) a phenotype overlapping spermatocytes in high proportion (50%) of the transfected cells, within 48 hours post transfection.

The protaminization of somatic cells might be extended to mammalian or not mammalian non-human animals, making the process reliable, with enormous advantages in fields like: non-human animal production and breeding, non-human transgenic animals, endangered non-human animals, and lastly in nuclear reprogramming for regenerative medicine.

Protaminized nuclei are physiologically remodeled by the oocyte, and successfully reprogrammed to the blastocyst stage.

It is therefore an object of the invention a method for reconstructing a non-human animal embryo, comprising the steps of:
a) Transiently expressing into a non-human donor cultivated cell at least a protein involved in male sperm maturation, or a synthetic or recombinant portion thereof able to confer to the chromatin of said cell a spermatozoa like chromatin conformation and
b) Identifying the nuclei wherein the chromatin has a spermatozoa like chromatin conformation, and
c) Transplanting the identified nuclei into a non-human animal enucleated oocyte to get reconstructed embryo and optionally
d) Culturing said reconstructed embryo in vitro
wherein the protein involved in male sperm maturation is protamine 1 or protamine 2.

The identification of the nuclei of step b) of the above method is preferably carried out by means of fluorescent tag linked protein or by fluorescence-activated cell sorting (FACS) or selecting the nuclei by morphological appearance (elongated structure).

Herein described is also the method according to the invention further comprising the step of:
e) Transferring said reconstructed embryo into a suitable non-human foster mother/shell.

Herein described is also a protein involved in male sperm maturation preferably belonging to the following group: very basic DNA-interacting non-histones proteins/peptides, transition proteins (TPS).

Preferably, said very basic DNA-interacting non-histones protein is protamine.

Preferably, said very basic DNA-interacting non-histones protein is protamine 1 or protamine 2.

Preferably the protamine 1 (also defined as Prm1) is human and has essentially the aa sequence present in NCBI database with the GenBank Accession No. AAA63249.1:
1 maryrccrsq srsryyrqrq rsrrrrrrsc qtrrramrcc rpryrprcrr h
   (SEQ ID NO:2), or has an amino acid sequence with a % identity not less than 70% of said aa protamine 1 sequence, or its mRNA is characterized by a cDNA sequence present in NCBI database with the Accession No. NM_002761.2:
(SEQ ID NO: 1) or its mRNA is characterized by a cDNA sequence with a % identity not less than 70% of said cDNA protamine 1 sequence.

In another preferred embodiment, protamine 2 is human and has essentially the aa sequence present in NCBI database with the GenBank Accession No.: CAA87066.1:
(SEQ ID NO:4) (Gene ID: 5620) or has an amino acid sequence with a % identity not less than 70% of said aa protamine 2 sequence, or its mRNA is characterized by a cDNA sequence present in NCBI database with the Accession No. NM_002762.3:
(SEQ ID NO: 3)
   or its mRNA is characterized by a cDNA sequence with a % identity not less than 70% of said cDNA protamine 2 sequence.

Preferably, said transition protein is transition protein 1 (TPS) and transition protein 2 (TPS).

More preferably, transition protein 1 (TPS) is human and has essentially the aa sequence present in NCBI database with the Accession No. NP_003275.1:
ORIGIN 1 mstsrklksh gmrrsksrsp hkgvkrggsk rkyrkgnlks rkrgddanrn yrshl //
   (SEQ ID NO:6), or has an amino acid sequence with a % identity not less than 70% of said aa transition protein 1 sequence,
   or its mRNA is characterized by a cDNA sequence present in NCBI database with the Accession No. NM_003284.3:
(SEQ ID NO:5)
   or its mRNA is characterized by a cDNA sequence with a % identity not less than 70% of said cDNA transition protein 1 sequence.

More preferably, transition protein 2 (TPS) is human and has essentially the aa sequence present in NCBI database with the Accession No. NP_005416.1:
1 mdtqthslpi thtqlhsnsq pqsrtctrhc qtfsqscrqs hrgsrsqsss qspashrnpt 61 gahsssghqs qspntspppk rhkktmnshh spmrptilhc rcpknrknle gklkkkkmak 121 riqqvyktkt rssgwksn
   (SEQ ID NO:8), or has an amino acid sequence with a % identity not less than 70% of said aa transition protein 2 sequence,
   or its mRNA is characterized by a cDNA sequence present in NCBI database with the Accession No.NM_005425.4:
(SEQ ID NO:7), or its mRNA is characterized by a cDNA sequence with a % identity not less than 70% of said cDNA transition protein 2 sequence.

In the instant invention, for % of identity (or % of similarity) it is intended the quantification of the % of elements equal (or similar) in a sequence of a biomolecule (Larkin et at., 2007). In the method according to the invention the non-human donor cultivated cell is preferably a somatic cell and/or fetal cell. Said somatic cell is a preferably a fibroblast. Other cells might be equally suitable.

Preferably, said non-human donor cultivated cell has been previously genetically modified.

Said genetic modification preferably includes the insertion of at least one heterologous DNA sequence and/or the deletion of at least one homologous gene and/or the modification of at least one homologous gene.

It is herein described that preferably said non-human donor cultivated cell and the non-human oocyte belong to the same species.

Alternatively, the non-human donor cultivated cell and the non-human oocyte may belong to different species.

Said species preferably belongs to the group of domestic animals, preferably farm, laboratory, and companion animals, and non mammalian species, preferably avian, amphibian, fish, reptiles.

Herein described is also a method for generating a non-human animal, comprising the steps of:
a) Transferring a reconstructed non-human animal embryo as above defined into a suitable non-human recipient animal or intermediate host or eggshell;
b) Causing said non-human animal embryo to develop to term;
d) Further breeding the resulting non-human animal.

The transfer of said reconstructed embryo into a suitable non-human recipient animal or intermediate host, may be an interspecific embryo transfer, i.e. the transfer of an embryo between a different species which is still able to carry the pregnancy to term.

By referring to eggshell it is referred to the application of this procedures to species with external reproduction, like avian/amphibians/reptiles eggshell.

Described herein is also a non-human animal obtainable by the method for generating a non-human animal as above defined and a non-human animal reconstructed embryo obtainable by the method for reconstructing a non-human animal embryo as above defined.

In the method of the invention, growing of reconstructed non-human embryos may be carried out to a stage compatible for their transfer into a suitable foster mother/shell.

In the context of the present invention, said synthetic or recombinant portion of the protein involved in male sperm maturation, e.g. of protamine 1, is e.g. from 7 to 25 aa long.

In the definition of "proteins involved in male sperm maturation", of "protamine 1", of "protamine 2", of "transition protein 1" or: of "transition protein 2" are comprised their allelic or orthologous variants, fragments, mutants, orthologues, derivatives or functional analogues. In the present invention, variants, fragments, mutants, orthologues, derivatives or functional analogues possess the same ability to confer to the chromatin of the donor cell a spermatozoa like chromatin conformation.

Preferably the variant or orthologs, derivatives and fragments thereof has at least one residue replaced by a different amino acid residue.

The variants of the present invention, obtained by technologies known in the art, are mutant proteins, which differ from the amino acid sequence of the wild type protein by the mutation of one or more single amino acid. In a very preferred embodiment of the present invention, only one amino acid replacement occurs on the sequence of the native protein. It is, however, encompassed by the subject of the present invention that the native protein can be further optimized by replacement of a plurality, e.g two or more, of amino acid replacements. The variants can therefore differ from the wild type protein sequence by amino acid replacements on 1-10, preferably 1, 2, 3, 4, 5 and 6 different amino acid target positions.

Moreover, the mutants or variants of the invention exhibit the same activity e.g. of protamine 1. The term "mutation" or "variant" as used in the context of the present invention can be understood as substitution, deletion and/or addition of single amino acid in the target sequence. Preferably, the mutation of the target sequence in the present invention is a substitution. The substitution can occur with different genetically encoded amino acid or by non-genetically encoded amino acids. Examples for non-genetically encoded amino acids are homocystein, hydroxyproline, omithin, hydroxylysine, citrulline, carnitine, etc.

As used herein, the term "orthologues" refers to proteins in different species than e.g. the proteins in Homo sapiens that evolved from a common ancestral gene by speciation. As an example of such orthologs, one can cite the proteins corresponding to protamine 1 in Mus musculus, Rattus norvegicus, Pan troglodytes, Bos Taurus, Gallus gallus, Xenopus laevis, Tetraodon nigroviridis, and Danio rerio.

As used herein, the term "derivatives" refers to polypeptides having a percentage of identity of at least 75% with the protein, e.g. with protamine 1, or orthologue thereof, preferably of at least 85%, as an example of at least 90%, and more preferably of at least 95%.

In the present invention, the transient expression of the protein involved in male sperm maturation or of the synthetic or recombinant portion thereof able to confer to the chromatin of said cell a spermatozoa like chromatin conformation, may be obtained by transfection, e.g. by Lipofectamine, with an expression vector, e.g. a plasmid, comprising the protein or portion thereof coding sequence. Said coding sequence may be under the control of a promoter able to efficiently express said coding sequence. Preferably the coding sequence is the sequence consisting essentially of the sequence of SEQ ID No. 1, 3, 5 or 7.

Transfection can be also carried out using calcium phosphate, by electroporation, by cell squeezing, by liposomes, electroporation, gene gun and other current methods of vectors delivery.

In the present invention, the uptake of protein involved in male sperm maturation or of the synthetic or recombinant portion thereof able to confer to the chromatin of said cell a spermatozoa like chromatin conformation may be obtained by electroporation, or other common cell poration techniques (streptolysin O, Protein uptake kit, etc).

In the context of the present invention it is referred to "reconstructed oocyte" or "reconstructed embryo" indifferently.

The nucleus of the donor cell may be genetically modified prior to the transfer in the recipient cell, in order to obtain non-human transgenic animals. The term "transgenic" refers to a non-human animal containing at least one gene from a different species in their somatic and germ line, and to a non-human animal whose germ line is subjected to technical intervention by recombinant DNA technology, as well.

The present invention will be illustrated by means of non-limiting examples referring to the following figures:

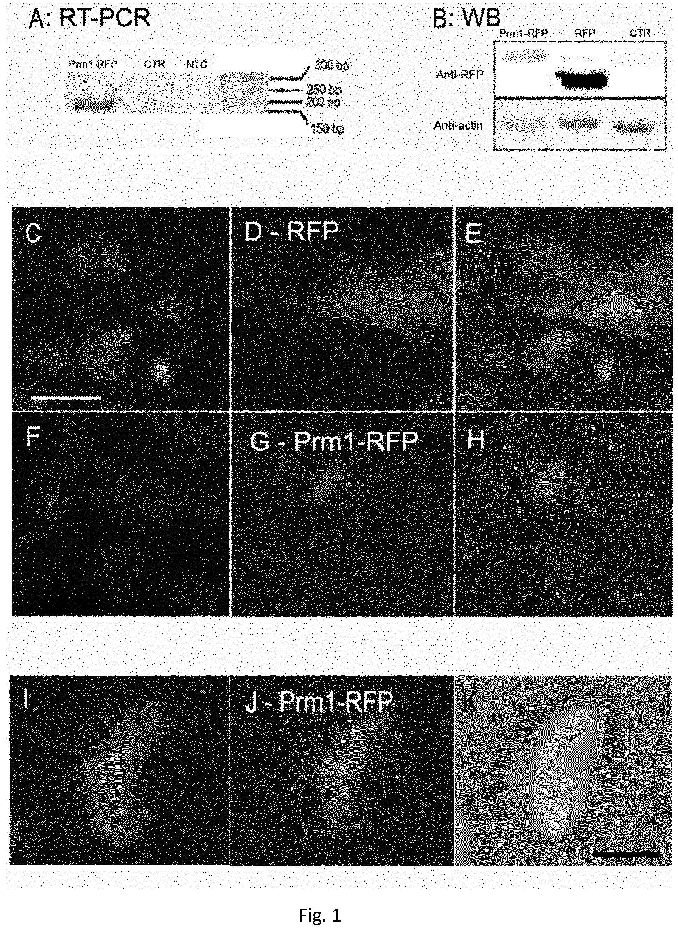

**Figure 1****. Expression of protamine 1 gene in adult fibroblasts** A - Protamine mRNA expression in sheep adult fibroblasts (RT-PCR). Prm1-RFP: fibroblasts transfected with protamine tagged with RFP. CTR: fibroblasts not transfected. NTC: no template control; B - Protamine protein expression in adult fibroblasts (Western blot, WB). Prm1-RFP: fibroblasts transfected with pPrm1-RFP. pTag-RFP: fibroblasts transfected with empty plasmid-RFP. CTR: fibroblasts not transfected. Immunoblotting with anti-tRFP, anti-actin were used as loading control; Tracking the RFP tag in cells transfected with pTag-RFP (C, D, E) and pPrm1-RFP (F, G, H); Nuclei (Hoechst stained), (C, F), RFP (D) or Prm1-RFP localizations (G), merge (E, H). Scale bar= 10 µm; Elongated nucleus in pPrm1-RFP transfected cell (I, J, K). Scale bar=10 µm

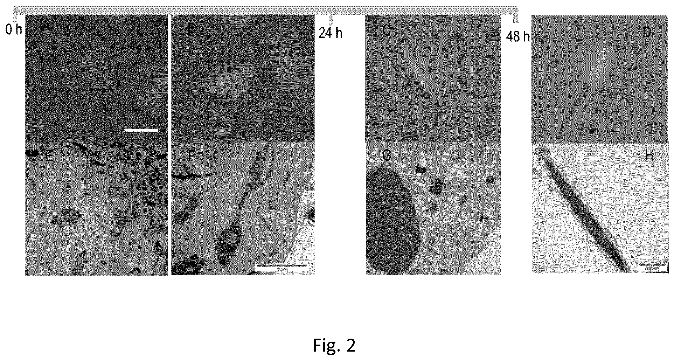

**Figure 2****. Timing of incorporation of protamine in somatic nuclei.** - Schematic representation of post transfection incorporation of protamine in somatic nuclei. pPrm1-RFP - protamine plasmide tagged with RFP; A - Nucleus of fibroblasts before the transcription of protamine; B - Incorporation of protamine in nucleus 16-20 h post transfection visible as spots; C - Complete incorporation of protamine in nucleus 40-48h post transfection, complete overlapping nucleus/protamine; E, F, G - Transmission electron microscope (TEM) analysis of adult sheep fibroblasts transfected with pPrm1-RFP; F- Nucleus of fibroblasts before the transcription of protamine., G - Nucleus of fibroblasts after 16-20 h post transfection. Visible partial compaction of chromatin; H- Nucleus of fibroblasts after 40 - 48h post transfection. Evident the complete chromatin compaction. Bars = 8 µm (A), 2 µm (F); D, H - Nucleus of spermatozoa stained with Hoechst (E) and analyzed by TEM (I). Scale bar: 500 nm

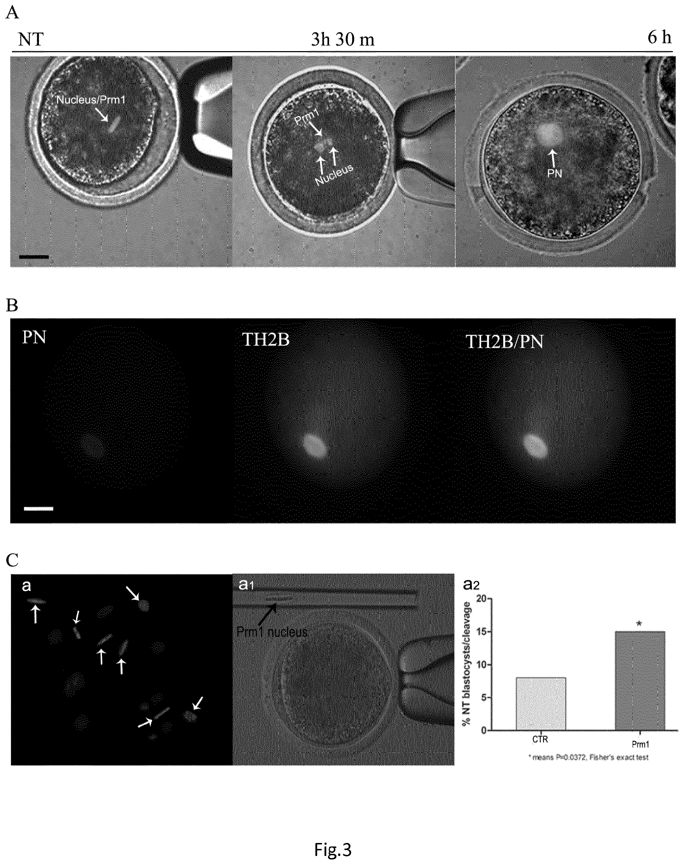

**Figure 3****. Nuclear remodelling of protaminized somatic fibroblasts.** A-Displacement protamine during pronucleus formation after Nuclear Transfer (NT); scale bar = 20 µm. Nucleus/Prm1 (overlapping), 3h 30m (displacement Prm1 from nucleus), 6h (protamine disappears and formation Pronucleus, PN); B- Incorporation TH2B in Pronucleus (PN) after NT of protaminized nuclei. Pronucleus (PN), TH2B , Merge (TH2B/PN). Scale bar = 20 µm; C - Somatic cell nuclear transfer (SCNT) of fibroblast transfected with pPrm1-RFP; a - Picture represents protamine (arrows) positive fibroblasts used as donors for SCNT; a1 - protaminized nucleus in injected capillar before nuclear transfer into enucleated MII sheep oocytes, a2 - Percentage of blastocysts produced by SCNT using control (CTR) and protaminized (Prm) sheep fibroblast as donor.

### EXAMPLE

### METHODS

### Plasmids construction

Sperm protamin 1 (Prm1) cDNA (accession N°: NM_002761.2 [SEQ ID NO:1]) was amplified from a human testis cDNA library with appropriate primes and cloned into a pTagRFP vector (Evrogen, Milan, Italy). The identity of the cloned cDNA and its in frame cloning C-terminal to RFP was verified by sequencing.

### Source of cells and transient transfection.

Sheep Adult Fibroblasts (SAF) were derived from ear biopsy of three female Sarda breed sheep (2 years old). Primary cultures were establishment from the biopsy and fibroblasts were used for transfection experiments between second and eighth passage. SAF were maintained during the culture in Dulbecco's modified eagle medium (DMEM) (Gibco, Life Technology, Milan, Italy) containing 10% FBS (Fetal Bovine Serum), 2mM Glutamine, 3.7 g/l NaHCO3 and 0.5% Gentamicin. SAF at 80% confluence were transfected with 3µg of pPrm1-RFP and pRFP (transfection CTR) (pTagRFP vector, FP141, Evrogen, Milan, Italy) by Lipofectamine 2000 (Invitrogen, Oslo, Norway), according to the manufacturer's instructions. The post-transfection medium was changed for DMEM containing 5nM Trichostatin A (TSA) 4h post transfection, and SAF were cultured for additional 16, 20, 40, 48 h.

Cells were cultured for 48 hours and checked for phenotypic changes following protamine expression.

The expression of protamine was additionally demonstrated by RT-PCR and western blotting analysis of transfected and control fibroblasts, as described below.

### RNA isolation and Reverse Transcription (RT-PCR)

Poly (A) + RNA was isolated from cells using Dynabeads mRNA DIRECT kit (Invitrogen Dynal AS, Oslo, Norway) according to the manufacturer's instructions. RT was performed using 80% of the eluted Poly (A) + RNA in a total volume of 20µl using the QuantiTect Reverse Transcription Kit (Qiagen, Milan, Italy). cDNAs were diluted 1:3 in H₂O. The PCR reactions were performed using the PCR Master Mix (Promega, Milano, Italy). PCR conditions: 95° 5' (95°C, 30", 58" 30°C, 72°C 30") x 35 cycles, 72°C 10 '. Primers were: FW; atggccagataccgatgct (SEQ ID NO: 9), RV; cagcatcttcgcctcctc (SEQ ID NO: 10); amplicon: 160bp.

### Western Blotting

Cell protein extracts were denatured by heating at 95°C for 5 min in 1% (v:v) sodium dodecyl sulphate (SDS), 1% (v:v) β-mercaptoethanol, 20% (v:v) glycerol in 50 mM Tris-HCl at pH 6.8. Samples were subjected to electrophoresis in 10% SDS polyacrylamide gels. After electrophoresis proteins were transferred to nitrocellulose membranes. Membranes were blocked in TBS-T (0.2% (v:v) Tween-20 in 20 mM Tris, 137 mM NaCl at pH 7.6) with 5% (w:v) skimmed milk, and then washed three times in TBS-T at room temperature. Membranes were incubated overnight (o/n) at 4°C with the primary antibody anti-tRFP (1:300, Evrogen, Milan, Italy) and anti-β-Actin as the loading control (1:1000; sc-1615, Santa Cruz Biotechnology, Santa Cruz, USA) diluted in blocking solution. After three washes with TBS-T, membranes were incubated for 1h at room temperature with the secondary antibody (anti-goat or anti-rabbit IgG HRP-labelled, Santa Cruz Biotechnology, Santa Cruz, USA) diluted 1:1000 in blocking solution. After three washes in TBS-T, the final detection was performed by enhanced chemiluminescence using the ECL Plus Western Blotting Detection System (Amersham-Pharmacia, Piscataway, USA). Image acquisition was carried out using the ChemiDoc System (Bio-Rad, Milan, Italy). Non transfected cells and transfected with only RFP tag (20 µg/lane) were used as positive controls.

### TEM analysis

Transfected cells were fixed and processed for TEM (Transmission Electron Microscopy) as described in the following. Cells were washed twice with PBS and fixed in glutaraldehyde (2.5% in 0.1 M cacodylate buffer, pH 7.4) for 24h. After washing in ddH₂O, cells were post-fixed in 2% OsO4 in ddH2O for 4h and washed three times in ddH₂O. Next, cells were dehydrated through a graded series of ethanol solutions (30% - 10min, 50% - 15min, 70% - 24h, 80% - 10min, 96% - 10min, 100% - 10min, acetone - twice for 15 min) and were infiltrated with graded concentrations of EPON resin in 100% acetone (1:3 - 20min, 1:1 - 24h, 3:1 - 2h), infused twice for 1 h in pure EPON resin and polymerized at 65°C for 24h. Next, 60 nm sections were prepared and examined using a LEO 912AB electron microscope. Images were captured using a Slow Scan CCD camera (Proscane) and EsiVision Pro 3.2 software (Soft Imaging Systems GmbH).

### Oocyte maturation

Sheep oocytes recovered from local ovine slaughterhouse were matured in vitro in bicarbonate-buffered TCM-199 medium (Gibco) (275mOsm) containing 2 mM glutamine, 100 mM cysteamine, 0.3 mM sodium pyruvate, 10% foetal bovine serum (FBS) (Gibco), 5 mg/ml FSH (Ovagen), 5 mg/ml LH, and 1 mg/ml estradiol in a humidified atmosphere of 5% CO2/air at 39°C for 24 h (Ptak G. et al, 2002).

### Nuclear transfer

Methods of in vitro embryo production were adapted from those previously described (Ptak et al., 2002). Oocytes were matured in vitro in a humidified atmosphere of 5% CO2/air at 39°C for 24 h. Oocytes were incubated in Hepes-buffered TCM-199 medium containing 4 mg/ml BSA, 7.5 mg/ml Cytochalasin B and 5 mg/ml Hoechst 33342 in an incubator for 15 minutes. Oocytes manipulation was carried out with a piezo-driven enucleation/injection pipette (PiezoXpert, Eppendorf). Enucleation was carried out in Hepes-buffered TCM-199 medium with 0.4% (w/v) BSA and Cytochalasin B with a Narishighe micromanipulator fitted to a Nikon Eclipse inverted microscope. No DNA vital dyes/UV irradiation was used to locate the chromosomes in the oocytes, but a blind aspiration of the cytoplasm surrounding the first polar body was conducted, and enucleation was confirmed later by Hoechst staining and UV irradiation of the aspired cytoplasmic fragments (Iuso et al., 2014). Enucleate oocytes were allowed to recover from the Cytochalasin B treatment and then directly injected with a nucleus, either from CTR or a Prm1-RFP fibroblasts suspended in PBS with 6% Polyvinylpyrrolidone (Sigma). Reconstructed oocytes were activated in Hepes-buffered TCM-199 medium containing 5 mg/ml Ionomycin for 5 minutes and then incubated in SOF medium plus antibiotics and 0.8% BSA containing 10 mM Dimethylaminopurine and 7.5 mg/ml Cytochalasin B for 3-5 hours and cultured for 10-12 hours in SOF enriched with 1% (v:v) minimum essential medium (MEM) nonessential amino acids (Gibco, Milan, Italy), 2% (v:v) basal medium Eagle (BME) essential amino acids, 1 mM glutamine, and 8 mg/ml BSA covered with mineral oil pre-washed in SOF. Cultures were checked for embryonic development every 24 hours till day 7 post activation.

### TH2B Immunostaining on pronuclear stage embryos

The zona pellucida of embryos at pronuclear stage (10-12 h after oocyte activation) was removed by incubation in 0.5% (w/v) pronase and acid Tyrode's solution for 30s. Embryos were then fixed in 4% PFA for 15 min and subjected to immunofluorescence analysis as described in Torres-Padilla et al. (2006) with the rabbit anti-TH2B antibody (1:700, ab23913 Abcam, UK). Finally, zygotes were mounted with VectaShield mounting medium with 5 µg/mL of DAPI.

### Statistical analysis

Fisher's exact test were used for comparing frequencies of transfection of somatic cells and development to blastocysts to enucleated oocytes injected with protaminized somatic cells. Probability values lower than 0.05 were considered significant. Statistical analyses were performed using GraphPad Prism 5.0 software.

### RESULTS

The transfected fibroblasts regularly expressed protamine (50%) in 34 replicates (Figure 1, A,B). Expression of mRNA and protein Prm1 was confirmed by RT-PCR, western blotting and by tracking the RFP-tag (Fig. 1A, B, G). Prm1-RFP co-localized with nuclei stained by Hoechst (Fig. IF, G, H) whereas in control, pRFP transfected fibroblasts, the red signal was diffused in cytoplasm and nucleus (Fig. 1C, D, E vs F, G, H). Prm1 translocate into the nuclei immediately after its translation and the end product of Prm1 assembly on somatic DNA is the acquisition of a nuclear morphology overlapping that found in elongating spermatids (Fig. 1I, J, K).

Protaminization started as focal points within the nucleus starting 10 hours post transfection, and induced radical nuclear transformation within 48 hours (Fig. 2), when a phenotype overlapping spermatocytes was acquired (Figure 2C, D). TEM analysis confirmed the extreme compaction of the nuclear compartment (Figure 2G).

It is concluded that the transient expression of protamine induces a radical chromatin reorganization of fibroblast nuclei (Fig. 2C, G), similar to that occurring in spermatozoa (Fig 2D,H).

Hence, the method of the present invention confers the chromatin of a somatic cell the structure of a spermatozoa DNA, thus perfectly compatible with the reprogramming machinery of the oocytes.

The remodeling of protaminized cells following nuclear transfer into enucleated oocytes was then analyzed. The protamine red tag was lost following oocyte activation, and the nuclei expand into a normal pronucleus (size: 16.2 ±2.3 µm) in 89% of the injected oocytes, Figure 3A. Meanwhile, protamine progressively disappeared in 77% of oocyte injected (28/36, Fig. 3A) and oocyte specific histones variant TH2B began to be assembled in the pronuclei (Fig. 3B). TH2B has recently described as the unique histone variant that plays a key role for nuclear reprogramming (Shinagawa et al., 2014), as it guides the protamine-histone chromatin transition post fertilization (Montellier et al., 2013).

The enucleated oocytes reconstructed with protaminized somatic nuclei were further cultured *in vitro* in 7 separated replicates, indicating their full competence to direct early embryonic cleavages. Moreover, the developmental frequencies to the blastocyst stage of Prm1 nuclei/derived embryos were significantly higher than control ones (NT blastocysts/cleavage: Prm1, 30/200; 15%; CTR, 14/176;7,9 %) (P=0.0372; Fisher's exact test, Fig. 3, a2).

The protamine-induced reprogramming method object of the present invention is a major breakthrough for nuclear reprogramming. Moreover, the simplicity and the robustness of the protocol developed by the present inventions render the method easily repeatable in all non-human animals reproducible through Somatic Cell Nuclear Transfer.

### References

Wells, D" Reprod Suppl. Vol. 61: 2003, 131-150.
Loi, P. et al. Nat Biotechnol. 2001, 19: 962-964
Holt et al., Reproduction 2004, 127: 317-324
Loi et al., PLOS ONE, 2008 vol. e297
Wakayama T. J Reprod Dev. 2007 53: 13-2.
Robl, et al., Theriogenology, 2007, 67: 127-133.
Niemann, and Kues: Reprod Fertil Dev, 2007, 19: 762-770.
Prather, et al., 'Theriogenology. 2003, 59: 115-23.
Wilmut I, et., Nature 1997, 385: 810-813
Loi P, Modlinski JA, Ptak G. In Principles of Cloning: Second Edition. Eds. Cibelli J., Wilmut I., Gurdon J. Elsevier . 2013, Pp 353-368.
Loi P, et al., Biol Reprod. 2002 Jul;67(1):126-32
Rousseaux S, et al., Reprod Biomed Online. 2008 Apr;16(4):492-503.
Rousseaux S, et al., Syst Biol Reprod Med. 2011 Feb;57(1-2):50-3.
Ptak, G et al;. Biol Reprod, 2002. 67, 1719-1725.
Iuso, D. et al., Cell Repr. 2013, 15(6), 490-494.
Larkin M.A., et al., (2007) Bioinformatics 23(21): 2947-2948.
Shinagawa, T. et al. Cell Stem Cell, 2014, 14(2):217-27.
Montellier, E., et al. Genes Dev. 2013. 27(15):1680-92.

## Claims

1. A method for reconstructing a non-human animal embryo, comprising the steps of:
a) Transiently expressing into a non-human donor cultivated cell at least a protein involved in male sperm maturation or a synthetic or recombinant portion thereof able to confer to the chromatin of said cell a spermatozoa like chromatin conformation and
b) Identifying the nuclei wherein the chromatin has a spermatozoa like chromatin conformation, and
c) Transplanting the identified nuclei into a non-human animal enucleated oocyte to get reconstructed embryo and optionally
d) Culturing said reconstructed embryo in vitro
wherein the protein involved in male sperm maturation is protamine 1 or protamine 2.

2. The method according to claim 1 wherein the non-human donor cultivated cell is a somatic cell and/or a fetal cell.

3. The method according to claim 2 wherein the somatic cell is a fibroblast.

4. The method according to anyone of previous claims, wherein said non-human donor cultivated cell has been previously genetically modified.

5. The method according to claim 4, wherein said genetic modification includes the insertion of at least one heterologous DNA sequence and/or the deletion of at least one homologous gene and/or the modification of at least one homologous gene.

## Patentansprüche

1. Verfahren zur Rekonstruktion eines nicht-menschlichen Tierembryos, das die folgenden Schritte umfasst:
a) transientes Exprimieren in einer kultivierten Zelle eines nicht-menschlichen Spenders mindestens eines Proteins, das an der Reifung männlicher Spermien beteiligt ist, oder eines synthetischen oder rekombinanten Teils davon, das in der Lage ist, dem Chromatin dieser Zelle eine spermatozoenartige Chromatinkonformation zu verleihen und
b) Identifizieren der Zellkerne, bei denen das Chromatin eine spermatozoenartige Chromatinkonformation aufweist,
und
c) Transplantieren der identifizierten Zellkerne in eine entkernte Eizelle eines nicht-menschlichen Tieres, um einen rekonstruierten Embryo zu erhalten und wahlweise
d) Kultivieren des rekonstruierten Embryos in vitro, wobei das an der Reifung männlicher Spermien beteiligte Protein Protamin 1 oder Protamin 2 ist.

2. Verfahren nach Anspruch 1, wobei die kultivierte nicht-menschliche Spenderzelle eine somatische Zelle und/oder eine fötale Zelle ist.

3. Verfahren nach Anspruch 2, wobei die somatische Zelle ein Fibroblast ist.

4. Verfahren nach einem der vorherigen Ansprüche, wobei die kultivierte nicht-menschliche Spenderzelle zuvor genetisch modifiziert wurde.

5. Verfahren nach Anspruch 4, wobei die genetische Modifizierung die Insertion von mindestens einer heterologen DNA-Sequenz und/oder die Deletion von mindestens einem homologen Gen und/oder die Modifizierung von mindestens einem homologen Gen umfasst.

## Revendications

1. Procédé de reconstruction d'un embryon animal non humain, comprenant les étapes suivantes :
a) expression temporaire dans une cellule cultivée de donneur non humain d'au moins une protéine impliquée dans la maturation du sperme mâle ou d'une partie synthétique ou recombinante de celui-ci pour conférer à la chromatine de ladite cellule une conformation de chromatine de type spermatozoïde et
b) identification des noyaux dans lesquels la chromatine a une conformation de chromatine de type spermatozoïde, et
c) transplantation des noyaux identifiés dans un oocyte énucléé d'animal non humain pour obtenir un embryon reconstruit et facultativement
d) culture dudit embryon reconstruit *in vitro* dans lequel la protéine impliquée dans la maturation du sperme mâle est la protamine 1 ou la protamine 2.

2. Procédé selon la revendication 1 dans lequel la cellule cultivée de donneur non humain est une cellule somatique et/ou une cellule foetale.

3. Procédé selon la revendication 2 dans lequel la cellule somatique est un fibroblaste.

4. Procédé selon l'une quelconque des revendications précédentes, dans lequel ladite cellule cultivée de donneur non humain a été préalablement génétiquement modifiée.

5. Procédé selon la revendication 4, dans lequel ladite modification génétique comprend l'insertion d'au moins une séquence d'ADN hétérologue et/ou la délétion d'au moins un gène homologue et/ou la modification d'au moins un gène homologue.
